# EUROPÄISCHE PATENTANMELDUNG

(11) **EP 2 431 914 A2**
(43) Veröffentlichungstag der Anmeldung: **21.03.2012**
(21) Anmeldenummer: 11007642.9
(22) Anmeldetag: 20.09.2011
(51) Int. Cl.: G06K 9/00, A61B 5/117

(54) **Anordnung und Verfahren zur Identifizierung der Echtheit von Fingerabdrücken**

(30) Priorität: 21.09.2010 DE 102010046785
(71) Anmelder: Technische Universität Dresden, 01069 Dresden (DE)
(72) Erfinder: Aust, Daniela, 01309 Dresden (DE); Baretton, Gustavo, 01326 Dresden (DE); Koch, Edmund, 01309 Dresden (DE); Roth, Michael, 01277 Dresden (DE); Steiner, Gerald, 08340 Schwarzenberg (DE)
(74) Vertreter: Hempel, Hartmut

(57) **Zusammenfassung**

Die Erfindung betrifft eine Anordnung (20) und ein Verfahren zur Identifizierung der Echtheit von Fingerabdrücken, aufweisend eine an eine datenverarbeitende Auswerteeinheit (10) angeschlossene Fingerabdruck-Aufnahmeeinheit (11), die mit einer Auflagefläche (21) eines Prismenkörpers (2) und mit Echtzeiterkennung für eine auf die Fingerabdruck-Aufnahmeeinheit (11) aufgelegten Fingerbeere (1) versehen ist, wobei der Fingerabdruck-Aufnahme- einheit (11) ein Sensor (4) nachgeordnet ist, um von der auf der Auflagefläche (21) aufgelegten Fingerbeere (1) Licht (3b) einer Beleuchtungseinrichtung (13) aufzunehmen, wobei der Sensor (4) signaltechnisch mit der Auswerteeinheit (10) verbunden ist, die mit einer nachgeordneten Anzeigeeinheit (12) in Verbindung steht. Dabei stellt der Prismenkörper (2) ein Prisma dar, dessen Grundfläche die Auflagefläche (21) für die Fingerbeere (1) ist und dessen eine geneigte Seitenfläche (22) die Eintrittsfläche für das einfallende infrarote Lichtbündel (3a) der Beleuchtungseinrichtung (13) und dessen andere geneigte Seitenfläche (23) die Austrittsfläche für das austretende infrarote Lichtbündel (3b) darstellen, wobei das einfallende und innenseitig des Prismas (2) auf die Grundfläche (21) auftreffende Lichtbündel (3a) und die Normale der Grundfläche (21) im Bereich eines Totalreflexionswinkels (α_{T}) liegen, wodurch das totalreflektierende Lichtbündel (3b) außenseitig des Prismas (2) auf der Grundfläche (21) ein evaneszentes elektromagnetisches Feld (3) ausbildet, das mit dem Abdruck der aufliegenden Fingerbeere (1) in Kontakt tritt und zellmolekularabhängig einer Absorption unterliegt, und danach als abgeschwächtes totalreflektiertes Lichtbündel (3b) auf einen Arraydetektor (4) zur molekülspektroskopischen Bildgebung geleitet wird, wobei die nachfolgend in der Auswerteeinheit (10) durchgeführte molekülspektroskopische Auswertung des abgeschwächten totalreflektierten infraroten Lichtbündels (3b) die Bewertung der biochemischen Zusammensetzung des Abdruckes gestattet, wobei als untersuchter Fingerabdruck der unmittelbare Kontakt der Fingerbeere (1) mit der Auflagefläche (21) oder der mittelbare Kontakt der Fingerbeere (1) als einen auf einem Material befindlichen, vorangegangenen Fingerabdruck mit der Auflagefläche (21) definiert ist.

## Beschreibung

Die Erfindung betrifft eine Anordnung und ein Verfahren zur Identifizierung der Echtheit von Fingerabdrücken, aufweisend eine an eine datenverarbeitende Auswerteeinheit angeschlossene Fingerabdruck-Aufnahmeeinheit, die mit einer Auflagefläche und mit Echtzeiterkennung für eine auf die Fingerabdruck-Aufnahmeeinheit aufgelegte Fingerbeere versehen ist, wobei zur Identifizierung und Echtzeiterkennung eine ortsaufgelöste molekülspektroskopische. Reflexionsmessung im Bereich von 4000 bis 500 Wellenzahlen durchgeführt wird, wobei der Fingerabdruck-Aufnahmeeinheit ein Sensor nachgeordnet ist, um von der auf der Auflagefläche, die durch eine Fläche eines Prismenkörpers gebildet wird, aufgelegten Fingerbeere abgelenktes Licht einer Beleuchtungseinrichtung aufzunehmen, wobei der Sensor signaltechnisch mit der Auswerteeinheit verbunden ist, die mit einer nachgeordneten Anzeigeeinheit in Verbindung steht.

In der Beschreibung wird durchgängig als Fingerabdruck der Abdruck der Fingerbeere eines Fingers bezeichnet.

Der menschliche Fingerabdruck ist einzigartig und wird daher häufig als sicherheitsrelevanter Indikator eingesetzt. Das Erkennen des Rillenmusters und dessen Indikation garantiert jedoch noch keine hinreichende Sicherheit vor Fälschungen. Kopien des Rillenmusters lassen sich vergleichsweise einfach z.B. durch Polymere und ähnliches Material herstellen. Ebenso ist bislang ungelöst die Identifizierung von Fingerabdrücken abgetrennter Finger oder ähnlicher Fälschungen mit natürlicher Haut.
Herkömmliche Verfahren zur Identifizierung der Echtheit von Fingerabdrücken gestatten diese nicht in entsprechend kurzer Zeit, nicht in situ oder online oder nicht mit der erforderlichen Empfindlichkeit.

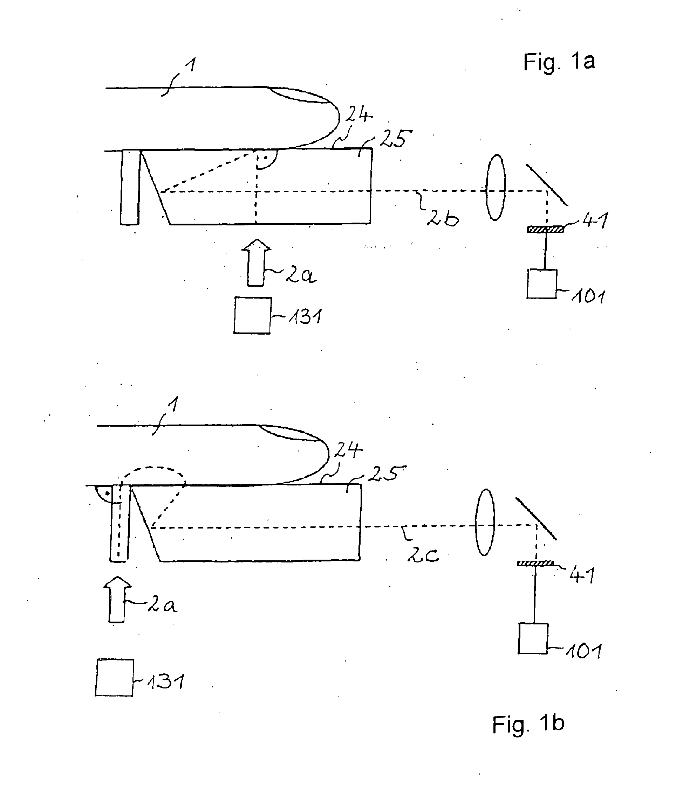

Ein in Fig. 1a dargestelltes Verfahren zur automatischen Aufnahme von Abdrücken eines Fingers ist in der Druckschrift US 2006/062438 A1 beschrieben, wobei das Verfahren mit einer an eine datenverarbeitende Auswerteeinheit 101 angeschlossenen, kamerabasierten Fingerabdruck-Aufnahmeeinheit mit einer Auflagefläche 24 und mit Echtzeiterkennung des auf die Fingerabdruck-Aufnahmeeinheit aufgelegten Fingers 1 versehen ist, wobei zur Echtzeiterkennung eine Mehrzahl von Reflexionsmessungen in einer vorgegebenen Mehrzahl von Reflexionswellenlängenbereichen durchgeführt wird. Der kamerabasierten Fingerabdruck-Aufnahmeeinheit ist ein Kamerasensor 41 zugeordnet, um von einem auf die Auflagefläche 24, die durch die Oberfläche eines Prismenkörpers 25 gebildet wird, aufgelegten Finger 1 gestreutes Licht 2b aus der Beleuchtungseinrichtung 131 mit Hilfe des in den Prismenkörper 25 einfallenden Lichtes 2a aufzunehmen. An den Kamerasensor 41 ist die Auswerteeinheit 101 angeschlossen.

Ein verbessertes Verfahren zur automatischen Aufnahme von Abdrücken eines Fingers mit einer an eine datenverarbeitende Auswerteeinheit 101 angeschlossenen Fingerabdruck-Aufnahmeeinheit ist in der Druckschrift EP 2 120 182 B1 beschrieben, wobei bei dem Verfahren neben den in Fig. 1 a dargestellten Reflexionsmessungen mindestens eine in Fig. 1 b dargestellte Transmissionsmessung in einem vorgegebenen Transmissionswellenlängenbereich von dem aufgelegten Finger 1 aufgenommen wird und die Transmissionsmessungen zusammen mit den unter senkrechter Einstrahlung von Licht 2a durchgeführten Reflexionsmessungen gemäß Fig. 1a auf Echtheitskriterien überprüft wird, die charakteristisch für sauerstoffreiches Blut sind, um durch diese Echtheitskriterien die Echtheit des aufgelegten Fingers 1 festzustellen. Wie in Fig. 1 b gezeigt ist, werden die Transmissionsmessungen durchgeführt, indem senkrecht eingestrahltes Licht 2a der Beleuchtungseinrichtung 131 in einem jenseits der Auflagefläche 24 liegenden Bereich des Fingers 1 in dessen der Auflagefläche 24 zugewandten Seite über den separaten Glasprismenkörper 25 eingestrahlt und das vom Finger 1 gestreute und aus dem Prismenkörper 25 austretende Licht 2c von dem Kamerasensor 41 aufgenommen wird.
Als Echtheitskriterium wird in der angeschlossenen Auswerteeinheit 101 überprüft, ob das Verhältnis der Absorptionsvermögen α₅₆₀₋₅₈₀/α₆₂₀₋₆₄₀ in festgelegten Wellenlängenbereichen 560nm-580nm und 620nm-640nm bei einem ersten leichten Andruck um einen vorgegebenen Faktor größer als das Verhältnis bei einem zweiten stärkeren Andruck ist. Diese Verfahrensweise erlaubt eine Unterscheidung zwischen einem echten Finger 1 und einem Leichenfinger. Aber zum Echtheitstest wird hier das Ausbleichen eines lebenden Fingers 1 bei höherem Andruck ausgenutzt.

Ein Problem besteht darin, dass es bei Auflage des Fingers schwierig ist zu entscheiden, was ein leichter Andruck und was ein stärkerer Andruck auf der Auflagefläche 24 ist und wie ein leichter Andruck und ein stärkerer Andruck gemessen werden. Außerdem wird nicht nur das letzte Fingerglied, die Fingerbeere, sondern ein wesentlich größerer Teil des Fingers 1 für den Echtheitstest auf den länglichen Prismenkörper 25 benötigt. Es werden in dem oben genannten verbesserten Verfahren auch zwei optisch verschiedene Messungen durchgeführt, was den Aufwand, auch in rechentechnischer Hinsicht erhöht. Bei beiden Messungen erfolgt die Beleuchtung mittels der Beleuchtungseinrichtung 131 des aufgelegten Fingers 1 immer senkrecht zur Auflagefläche 24. Gemessen wird von der Kamera 41 jeweils das gestreute Licht.

Der Erfindung liegt deshalb die Aufgabe zugrunde, eine Anordnung und ein Verfahren zur Identifizierung der Echtheit von Fingerabdrücken anzugeben, die derart geeignet ausgebildet sind, die Echtheit als sicherheitsrelevantes Merkmal zu überprüfen und Fälschungen zu erkennen. Außerdem sollen sich die Fingerabdrücke von lebenden Personen eindeutig identifizieren lassen und zwar sowohl hinsichtlich der biochemischen Merkmale als auch bezüglich des Rillenmusters.

Die Aufgabe der Erfindung wird durch die Merkmale der Patentansprüche 1 und 6 gelöst.
Die Anordnung zur Identifizierung der Echtheit von Fingerabdrücken, aufweisend eine an eine datenverarbeitende Auswerteeinheit angeschlossene Fingerabdruck-Aufnahmeeinheit, die mit einer Auflagefläche und mit Echtzeiterkennung für eine auf die Fingerabdruck-Aufnahmeeinheit aufgelegte Fingerbeere versehen ist, wobei zur Identifizierung und Echtzeiterkennung eine ortsaufgelöste molekülspektroskopische Reflexionsmessung im Bereich von 4000 bis 500 Wellenzahlen durchgeführt wird, wobei der Fingerabdruck-Aufnahmeeinheit ein Sensor nachgeordnet ist, um von der auf der Auflagefläche, die durch eine Fläche eines Prismenkörpers gebildet wird, aufgelegten Fingerbeere abgelenktes Licht einer Beleuchtungseinrichtung aufzunehmen, wobei der Sensor signaltechnisch mit der Auswerteeinheit verbunden ist, die mit einer nachgeordneten Anzeigeeinheit in Verbindung steht,
wobei der Prismenkörper ein Prisma darstellt, dessen Grundfläche die Auflagefläche für die Fingerbeere ist und dessen eine geneigte Seitenfläche die Eintrittsfläche für das einfallende infrarote Lichtbündel der Beteuchtungseinrichtung und dessen andere geneigte Seitenfläche die Austrittsfläche für das austretende infrarote Lichtbündel darstellen, wobei das einfallende und innenseitig des Prismas auf die Grundfläche auftreffende Lichtbündel und die Normale der Grundfläche im Bereich eines Totalreflexionswinkels α_{T} liegen, wodurch das totalreflektierende Lichtbündel außenseitig des Prismas auf der Grundfläche ein evaneszentes elektromagnetisches Feld ausbildet, das mit dem Abdruck der aufliegenden Fingerbeere in Kontakt tritt und zellmolekularabhängig einer Absorption unterliegt, und danach als totalreflektiertes Lichtbündel auf einen den Sensor darstellenden Arraydetektor zur spektroskopischen Bildgebung geleitet wird, wobei gemäß dem Kennzeichenteil des Patentanspruchs 1
der Bildkontrast auf der molekularen Zusammensetzung der Fingerbeere beruht, wobei die molekülspektroskopische Bildgebung auf den registrierten Absorptionsbereichen aus den aufgenommenen Molekülspektren basiert, wobei die Molekülspektren von der Ausbildung des evaneszenten elektromagnetischen Feldes und dessen Absorption in Abhängigkeit von der vorhandenen molekularen Struktur der Zellen geprägt werden, wobei in der Auswerteeinheit die Molekülspektren im Wellenzahl-Bereich zwischen 1600cm⁻¹ und 1780cm⁻¹ auf Proteine, freie Carbonylgruppen und Fettsäuren der Zellen von verschiedenen Fingerabdrücken bezogen und verglichen werden, wobei aus dem Vergleich der unterschiedlichen spektralen Muster der Proteine, der Carbonylgruppen und Fettsäuren und aus deren Spaltung und Abbau Indizien zur Unterscheidung der Fingerabdrücke gegeben sind, die durch den Proteinabbau, den Fettsäurenabbau und die Abbauprodukte der Carbonylgruppen unterstützt werden, wobei die spektralen Muster die Gesamtheit der Absorptionssignale einer Stoffgruppe oder Molekülgruppe darstellen, wobei die in der Auswerteeinheit durchgeführte molekülspektroskopische Auswertung des abgeschwächten totalreflektierten infraroten Lichtbündels die Bewertung der biochemischen Zusammensetzung des Fingerabdruckes gestattet und wobei die Auswerteeinheit zumindest folgende Funktionseinheiten, in der folgenden Reihenfolge auch hardwaremäßig miteinander verbunden, enthält:
- eine erste Funktionseinheit zur Datenvorbehandlung und Entfernen von Störungen,
- eine zweite Funktionseinheit für eine multivariate Datenanalyse und Ermittlung in einem signifikanten zellmolekularabhängigen Absorptionsbereich mit dem Wellenzahlbereich zwischen 1600cm⁻¹ und 1780cm⁻¹ ,
- eine dritte Funktionseinheit zur Speicherung einer Tabelle mit dem signifikanten Absorptionsbereich mit dem Wellenzahlbereich zwischen 1600cm⁻¹ und 1780cm⁻¹ mit dessen Position und mit dessen Intensität als Tabellenparameter,
- eine Vergleichseinheit zum Vergleich der Datensätze des aktuellen Fingerabdrucks mit den abgerufenen gespeicherten Referenz-Spektren im Wellenzahlbereich zwischen 1600cm⁻¹ und 1780cm⁻¹ aus der Datenbank und zur Erzeugung von Vergleichsergebnissen,
- eine Klassifizierungseinheit zur Klassifizierung der weitergeleiteten Vergleichsergebnisse mit Klassifizierungsergebnissen als identifizierter übereinstimmender Fingerabdruck oder als Fälschung und zur Übertragung der Klassifizierungsergebnisse an die der Auswerteeinheit nachgeordnete Anzeigeeinheit.

Die Molekülspektren können in Form von Extinktions(E)-Wellenzahl(v)-Kurven (E(v)) oder Transmissionsspektren oder Absorptionsspektren ausgebildet sein.

Der dem Arraydetektor angeschlossenen Auswerteinheit sind eine Datenbank, in der Referenz-Spektren gespeichert sind und die zu Vergleichen mit Datensätzen eines aktuellen Fingerabdrucks zur Verfügung stehen, und eine Speichereinheit, die für die Speicherung der Datensätze von selektierten Spektren des aktuellen Fingerabdrucks dient, zugeordnet, wobei die Datensätze eines aktuellen Fingerabdrucks die biochemischen Informationen und die Zuordnung zur örtlichen Position des zugehörigen Rillenmusters enthalten.

Die Datenbank, die Speichereinheit und die in der Auswerteeinheit enthaltenen Funktionseinheiten sowie die Vergleichseinheit und die Klassifizierungseinheit der Auswerteeinheit sind Hardware-Baugruppen, die zumindest mit funktionsbedingten und -bezogenen programmtechnischen Mitteln versehen sind und untereinander in signaltechnischer und programmtechnischer Verbindung stehen können.
Die in der Datenbank gespeicherten Referenz-Spektren können von
- Vorab-Fingerabdrücken mit der Haut von lebenden Fingern,
- Vorab-Fingerabdrücken von teilgeschädigten und totalgeschädigten Hautzellen der Finger,
- Vorab-Abdrücken in Materialen mit nachgeahmten künstlichen Fingerabdrücken (wie bei Gelatine oder Holzleim),
- Vorab-Abdrücken von realen Fingerabdrücken auf Materialien (Leder, Textilien, Metall) sowie
- unbedruckten Materialien
   sein, die für die Vergleiche in der Auswerteeinheit aus der Datenbank abgefragt werden können.

Das Verfahren zur Identifizierung der Echtheit von Fingerabdrücken kann durchgeführt werden mit einer an eine datenverarbeitenden Auswerteeinheit angeschlossenen Fingerabdruck-Aufnahmeeinheit, die mit einer Auflagefläche und mit Echtzeiterkennung für eine auf die Fingerabdruck-Aufnahmeeinheit aufgelegte Fingerbeere versehen ist, wobei zur Echtzeiterkennung eine Reflexionsmessung in einem vorgegebenen Wellenzahlbereich durchgeführt wird, wobei der Fingerabdruck-Aufnahmeeinheit ein Sensor nachgeordnet ist, um von der auf die Auflagefläche, die durch eine Fläche eines Prismenkörpers gebildet wird, aufgelegten Fingerbeere abgelenktes Licht einer Beleuchtungseinrichtung aufzunehmen, wobei der Sensor signaltechnisch mit der Auswerteeinheit verbunden ist, die mit einer nachgeordneten Anzeigeeinheit in Verbindung steht, unter Einsatz der vorgenannten Anordnung,
wobei gemäß dem Kennzeichenteil des Patentanspruchs 6 folgende Schritte durchgeführt werden:
- Vorab-Speicherung von selektierten Referenz-Spektren in einer Datenbank
   und
   während und nach Aufnahme des aktuellen Fingerabdrucks
- Speicherung von selektierten Spektren eines aktuellen Fingerabdrucks als Datensätze in einer Speichereinheit,
- Datenvorbehandlung und Entfernen von Störungen in den Datensätzen des aktuellen Fingerabdrucks aus der Speichereinheit,
- Multivariate Datenanalyse und Ermitteln von signifikanten Absorptionsbereichen aus den Datensätzen des aktuellen Fingerabdrucks,
- Speicherung von Tabellen mit den signifikanten Absorptionsbereichen, deren Positionen und deren Intensitäten als Tabellenparameter,
- Vergleich zwischen den in der Datenbank enthaltenen Referenz-Spektren nach deren Abfrage und den in den Tabellen vorhandenen Tabellenparametern der Datensätze des aktuellen Fingerabdrucks nach Übermittlung,
- Klassifizierung der Vergleichsergebnisse in mit den Referenz-Spektren übereinstimmende Fingerabdrücke und in mit den Referenz-Spektren nicht übereinstimmende Fingerabdrücke, die als Fälschung definiert werden, als Klassifizierungsergebnisse sowie
- Übergabe der Klassifizierungsergebnisse als spektroskopische Rillenmuster und Extinktions-Wellenzahl-Kurven E(v) zur Anzeige.

Als signifikante Absorptionsbereiche können ausgewählte Absorptionsbanden als solche, Kanten, Flanken, Schultern von Absorptionsbanden oder zusammengesetzte Absorptionsbanden gelten.

In der Endphase der Verfahrensschritte bei dem Vergleich und bei der Klassifizierung des aktuellen Fingerabdrucks werden folgende detaillierte Unterschritte in Verbindung mit den vorab genannten hauptsächlichen Schritten durchgeführt:
- Unterschritt:
   Erkennen von Pixeln der aus der Tabelle der Funktionseinheit bereitgestellten Pixel mit chemischem Informationsgehalt über die zwischenliegenden bearbeiteten hauptsächlichen Schritte,
- Unterschritte:
   Abarbeitung eines in die Vergleichseinheit eingebundenen ersten Vergleichsalgorithmus zur Feststellung eines aktuellen Fingerabdrucks nach Abfrage von Referenz-Spektren und Abarbeitung eines mit dem ersten Vergleichsalgorithmus in Verbindung stehenden zweiten Vergleichsalgorithmus zur Materialunterschiedsfeststellung des aktuellen Fingerabdrucks
   mit folgenden
- Unterschritten:
   - erste Entscheidung auf Echtheit des aktuellen Fingerabdrucks,
   - zweite Entscheidung auf Fälschung des aktuellen Fingerabdrucks und
   - dritte Entscheidung auf Materialfälschung am aktuellen Fingerabdruck, sowie
- Schritt:
   Übertragung einer der zutreffenden Entscheidungen zur Anzeigeeinheit.

Die molekülspektroskopische Bildgebung kann auf den registrierten Absorptionsbereichen aus den aufgenommenen Extinktionsverläufen E(v) in einem Extinktions(E)-Wellenzahl(v)-Koordinatensystem beruhen, wobei die Extinktionsverläufe E(v) auf der Ausbildung des evaneszenten elektromagnetischen Feldes und dessen Schwächung durch elektromagnetische Absorption in Abhängigkeit von der vorhandenen molekularen Struktur der Zellen geprägt werden, wobei das evaneszente elektromagnetische Feld durch die Totalreflexion an der Grundfläche/Auflagefläche des Primas zwischen Glas und Luft entsteht.

Die Molekülspektren, insbesondere die Extinktions(E)-Wellenzahl(v)-Kurven werden hauptsächlich auf Proteine, freie Carbonylgruppen und Fettsäuren der Zellen von verschiedenen Fingerabdrücken mit intakten (lebenden) Zellen, abgestuft teilgeschädigten Zellen oder total geschädigten (toten) Zellen bezogen, wobei der Unterschied zwischen lebenden Zellen und toten Zellen durch die unterschiedlichen E(v)-Kurven, insbesondere den spektralen Mustern der Proteine, der Carbonylgruppen und Fettsäuren durch deren Spaltung und Abbau gegeben sind, wobei der Proteinabbau ein Indiz für die toten Zellen des aktuellen Fingerabdrucks gibt, wobei der Fettsäurenabbau bei toten Zellen eines Fingers größer als bei lebenden Zellen ist und ebenso die Abbauprodukte der Carbonylgruppen bei toten Fingern größer als bei lebenden Zellen eines Fingers sind, so dass die Extinktionswerte E_{T}(v) der toten Zellen bei Fingern geringer als die Extinktionswerte E_{L}(v) der lebenden Zellen bei Fingern sind.

Unter einem spektralen Muster wird die Gesamtheit der Absorptionssignale einer bestimmten Stoffgruppe oder Molekülgruppe verstanden.

Die Vorteile der Erfindung bestehen darin, dass die infrarot-molekülspektroskopische Bildgebung die Aufnahme von spektroskopischen Bildern innerhalb von wenigen Sekunden gestattet. Anhand der Spektren lässt sich die biochemische Komposition des Fingerabdruckes erkennen. Die laterale Verteilung bestimmter Merkmale zeigt das typische Rillenmuster. Fingerabdrücke von technischen Materialien sind vergleichsweise einfach von echten Fingerabdrücken zu unterscheiden.
Der biochemische Fingerabdruck eines toten Fingers (z.B. von einer Leiche) zeigt zwar eine hohe Ähnlichkeit zu dem Fingerabdruck einer Fingerbeere einer lebenden Person, aber zugleich auch deutliche Unterschiede. Abbauprodukte von Proteinen und Lipiden treten bei einem toten Finger deutlich hervor. Anhand dieser molekularen Zusammensetzung lässt sich der tote Finger als eine Fälschung erkennen.

Weiterbildungen und weitere Ausgestaltungen der Erfindung sind in mehreren Unteransprüchen angegeben.

Die Erfindung wird anhand von Ausführungsbeispielen und mittels Zeichnungen näher erläutert.
Es zeigen:
- Fig. 1: schematische Darstellungen der Aufnahme des Fingerabdrucks zur spektroskopischen identifizierung über das Verhältnis von Absorptionsvermögen in verschiedenen festgelegten Wellenlängenbereichen nach dem Stand der Technik, wobei Fig. 1a eine Anordnung zur Reflexionsmessung in einer Fingerabdruck-Aufnahmeeinheit und
Fig. 1 b eine Anordnung zur Transmissionsmessung in einer Fingerabdruck-Aufnahmeeinheit zeigen,
- Fig. 2: schematische Darstellungen einer erfindungsgemäßen Anordnung zur Identifizierung der Echtheit von Fingerabdrücken mit einer Fingerabdruck-Aufnahmevorrichtung, wobei
Fig. 2a eine schematische Darstellung der Anordnung,
Fig. 2b eine perspektivische Darstellung der Fingerabdruck-Aufnahmevorrichtung und
Fig. 2c eine Darstellung der Fingerabdruck-Aufnahmevorrichtung im Schnitt zeigen,
- Fig. 3: ein Flussdiagramm des Verfahrens zur Identifizierung der Echtheit von Fingerabdrücken,
- Fig. 4: eine Darstellung der Endphase der Verfahrensschritte des Vergleiches und der Klassifizierung des aktuellen Fingerabdrucks,
- Fig. 5: eine Bearbeitungsfolge der aufgenommenen Rohdaten, wobei
Fig. 5a eine Darstellung von Rohdaten der molekülspektroskopischen Bildgebung,
Fig. 5b eine Darstellung der molekülspektroskopischen Bildgebung von vorbehandelten Daten durch Eliminierung von Pixeln und durch Korrektur von Artefakten,
Fig. 5c eine aus einem aufgenommenen Molekülspektrum ausgebildete Extinktions(E)-Wellenzahl(v)-Kurve eines Mittelspektrums der selektierten Pixel ,
zeigen,
- Fig. 6: Ausschnitte von Fingerabdrücken, wobei Fig. 6a einen Ausschnitt des Fingerabdrucks eines toten Fingers und Fig. 6b einen Ausschnitt des Fingerabdruck eines lebenden Fingers zeigen,
- Fig. 7: eine aus einem aufgenommenen Molekülspektrum ausgebildete Extinktions(E)-Wellenzahl(v)-Kurve in Bezug auf Proteine, freie Carbonylgruppen und Fettsäuren der Zellen im relevanten Bereich der Fingerbeere,
- Fig. 8: ein erstes Beispiel der Anzeige für Gelatine als Fälschung in Bezug auf Rillenmuster und ausgewerteter spektroskopischer Bildgebung, wobei
Fig. 8a den zugehörigen gefälschten Fingerabdruck aus getrockneter Gelatine mit Rillenmuster und Fig. 8b die zugehörige ausgewertete Extinktions(E)-Wellenzahl(v)-Kurve zeigen,
- Fig. 9: ein zweites Beispiel der Anzeige für Holzleim als Fälschung in Bezug auf Rillenmuster und ausgewerteter spektroskopischer Bildgebung, wobei
Fig. 9a den zugehörigen gefälschten Fingerabdruck aus getrocknetem Holzleim mit Rillenmuster und
Fig. 9b die zugehörige ausgewertete Extinktions(E)-Wellenzahl(v)-Kurve zeigen.

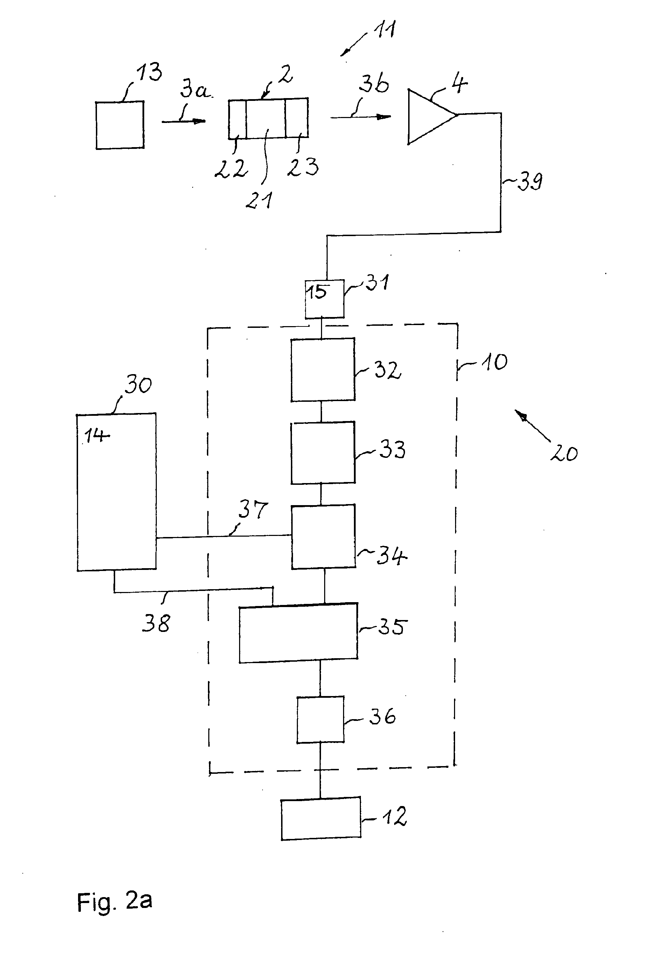

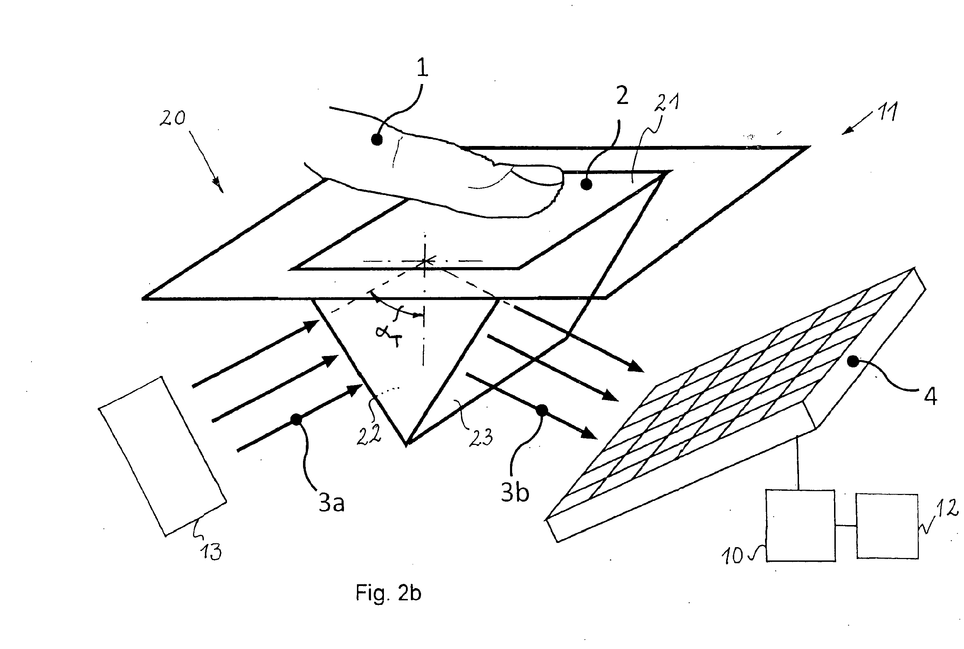

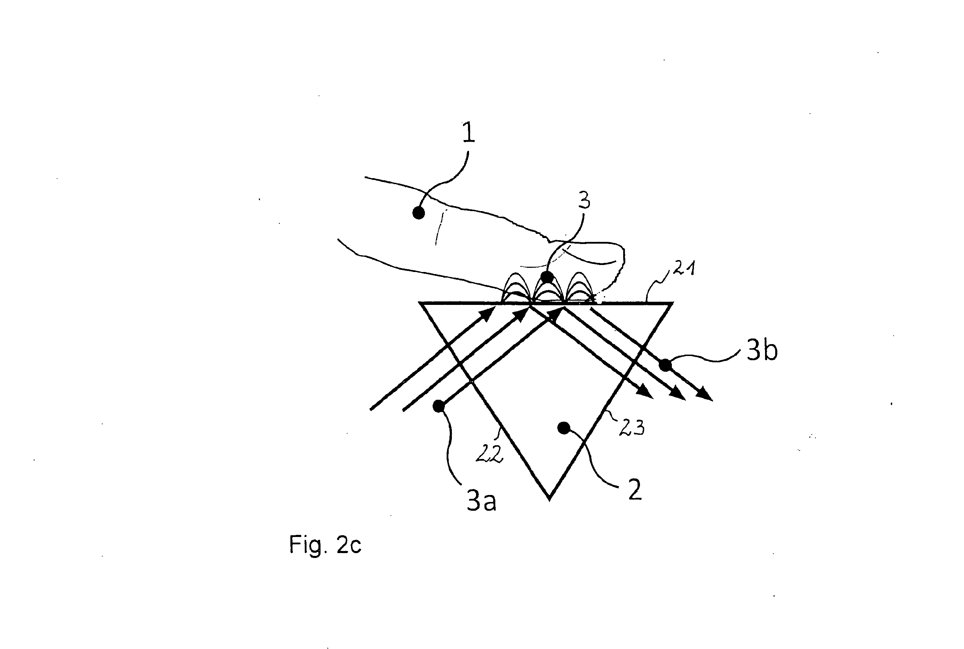

Im Folgenden werden Fig. 2a, 2b und 2c gemeinsam betrachtet.
In Fig. 2a ist eine schematische Darstellung einer erfindungsgemäßen Anordnung 20 zur Aufnahme eines Fingerabdrucks zur spektroskopischen Identfizierung auf Echtheit gezeigt. Die Anordnung 20 weist eine an eine datenverarbeitende Auswerteeinheit 10 angeschlossene, kamerabasierte Fingerabdruck-Aufnahmeeinheit 11 auf, die mit einer Auflagefläche 21 und mit Echtzeiterkennung für eine auf die Fingerabdruck-Aufnahmeeinheit 11 aufgelegten Fingerbeere 1, wie in den Fig. 2b, 2c gezeigt ist, versehen ist, wobei zur Echtzeiterkennung eine Reflexionsmessung in einem vorgegebenen Wellenzahlbereich durchgeführt wird, wobei der kamerabasierten Fingerabdruck-Aufnahmeeinheit 11 ein Pixelfeld 4 der Kamera nachgeordnet ist, um von der auf die Auflagefläche 21, die durch eine Fläche eines Prismenkörpers 2 gebildet wird, aufgelegten Fingerbeere 1 abgelenktes Licht 3b einer Beleuchtungseinrichtung 13 aufzunehmen, wobei das Pixelfeld 4 der Kamera signaltechnisch mit der Auswerteeinheit 10 verbunden ist, die mit einer nachgeordneten Anzeigeeinheit 12 in Verbindung steht.

Der Prismenkörper 2 stellt in Fig. 2b, Fig. 2c ein im Querschnitt dreieckförmiges Prisma dar, dessen Grundfläche die Auflagefläche 21 für die Fingerbeere 1 ist und dessen eine geneigte Seitenfläche 22 die Eintrittsfläche für das einfallende infrarote Lichtbündel 3a der Beleuchtungseinrichtung 13 und dessen andere geneigte Seitenfläche 23 die Austrittsfläche für das austretende infrarote Lichtbündel 3b darstellen, wobei das einfallende und innenseitig des Prismas 2 auf die Grundfläche 21 auftreffende Lichtbündel 3a und die Normale der Grundfläche 21 im Bereich eines Totalreflexionswinkels α_{T} liegen, wodurch das totalreflektierende Lichtbündel 3b außenseitig des Prismas 2 auf der Grundfläche 21, die dort gleich der Auflagefläche ist, ein evaneszentes elektromagnetisches Feld 3 ausbildet, das mit dem Abdruck der aufliegenden Fingerbeere 1 in Kontakt tritt und zellmolekularabhängig einer Schwächung durch Absorption unterliegt, und danach als abgeschwächtes totalreflektiertes Lichtbündel 3b auf einen als Pixelfeld 4 ausgebildeten Arraydetektor geleitet wird, wobei die nachfolgend in der Auswerteeinheit 10 durchgeführte molekülspektroskopische Auswertung des abgeschwächten totalreflektierten infraroten Lichtbündels 3b die Bewertung der biochemischen Komposition des Abdrucks der Fingerbeere 1 gestattet.

Als untersuchter Fingerabdruck kann z.B. sowohl der unmittelbare Kontakt der Fingerbeere 1 mit der Auflagefläche 21 als auch der mittelbare Kontakt der Fingerbeere 1 als einen auf einem Material befindlichen, vorangegangenen Fingerabdruck mit der Auflagefläche 21 definiert sein.

Als Prisma 2 kann ein dreiseitiges Prisma, ein gleichschenkliges Prisma, ein ungleichschenkliges Prisma oder auch ein trapezartiges Prisma eingesetzt werden.

Der dem Arraydetektor 4 angeschlossenen Auswerteinheit 10 ist eine Datenbank 30, in der Referenz-Spektren 14 gespeichert sind und die zu Vergleichen mit Datensätzen 15 eines aktuellen Fingerabdrucks zur Verfügung stehen, und eine Speichereinheit 31, die für die Speicherung der Datensätze 15 von selektierten Spektren des aktuellen Fingerabdrucks der Fingerbeere 1 dient, zugeordnet, wobei die Datensätze 15 eines aktuellen Fingerabdrucks die biochemischen Informationen und die Zuordnung zur örtlichen Position des zugehörigen Rillenmusters enthalten.

Die Auswerteeinheit 10 enthält zumindest folgende Funktionseinheiten:
- erste Funktionseinheit 32 zur Datenvorbehandlung und Entfernen von Störungen,
- zweite Funktionseinheit 33 für eine multivariate Datenanalyse und Ermittlung von signifikanten zellmolekularabhängigen Absorptionsbereichen,
- dritte Funktionseinheit 34 zur Speicherung einer Tabelle mit den signifikanten Absorptionsbereichen, zur Speicherung der Position und der Intensität der Absorptionsbereiche,
- Vergleichseinheit 35 zum Vergleich der Datensätze 15 des aktuellen Fingerabdrucks mit den abgerufenen gespeicherten Referenz-Spektren 14 aus der Datenbank 30 und zur Erzeugung von Vergleichsergebnissen,
- Klassifizierungseinheit 36 zur Klassifizierung der weitergeleiteten Vergleichsergebnisse mit Klassifizierungsergebnissen als identifizierter übereinstimmender Fingerabdruck oder als Fälschung und zur Übertragung der Klassifizierungsergebnisse an die der Auswerteeinheit 10 nachgeordnete Anzeigeeinheit 12.

Als signifikante Absorptionsbereiche können Absorptionsbanden als solche, Kanten, Flanken, Schultern von Absorptionsbanden oder zusammengesetzte Absorptionsbanden gelten.

Die in der Datenbank 30 gespeicherten Referenz-Spektren 14 können von
- Vorab-Fingerabdrücken durch die Haut von lebenden Fingerbeeren 1,
- Vorab-Fingerabdrücken von teilgeschädigten und totalgeschädigten Hautzellen der Fingerbeeren 1,
- Vorab-Abdrücken in Materialen mit nachgeahmten künstlichen Fingerabdrücken (wie bei Gelatine oder Holzleim) sowie
- Vorab-Abdrücken von realen Fingerabdrücken auf Materialien (Leder, Textilien, Metall) oder
- unbedruckten Materialien
sein, die für die Vergleiche in der Auswerteeinheit 10 aus der Datenbank 30 abgefragt werden können.

Die Datenbank 30, die Speichereinheit 31 und die Funktionseinheiten 32, 33, 34, die Vergleichseinheit 35 und die Klassifizierungseinheit 36 der Auswerteeinheit 10 können Hardware-Baugruppen sein, die zumindest mit funktionsbedingten programmtechnischen Mitteln versehen sind und untereinander in signaltechnischer und programmtechnischer Verbindung stehen können.

Durch die mit programmtechnischen Mitteln versehenen Funktionseinheiten 32, 33,34,35,36 der Auswerteeinheit 10 lässt sich die Echtheit oder Fälschung eines Fingerabdrucks erkennen.

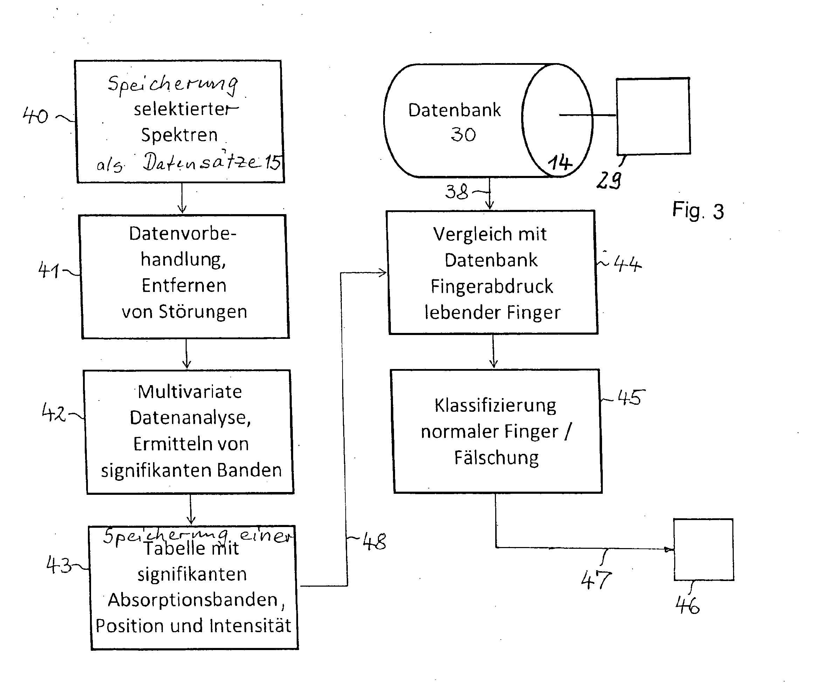

In Fig. 3 ist ein Flussdiagramm des Verfahrens zur Identifizierung der Echtheit von Fingerabdrücken 15 unter Einsatz der vorgenannten Vorrichtung 20 dargestellt, wobei das Verfahren folgende wesentliche Schritte aufweist:
- Vorab-Speicherung 29 von selektierten Referenz-Spektren 14 in einer Datenbank 30 und
   während und nach Aufnahme des aktuellen Fingerabdrucks in dieser Reihenfolge
- Speicherung 40 von selektierten Spektren des aktuellen Fingerabdrucks als Datensätze 15 in einer Speichereinheit 31,
- Datenvorbehandlung 41 und Entfernen von Störungen in den Datensätzen 15 des aktuellen Fingerabdrucks aus der Speichereinheit 31,
- Multivariate Datenanalyse 42 und Ermitteln von signifikanten Absorptionsbereichen aus den Datensätzen 15 des aktuellen Fingerabdrucks,
- Speicherung 43 von Tabellen mit den signifikanten Absorptionsbereichen, deren Positionen und deren Intensitäten als Tabellenparameter,
- Vergleich 44 zwischen den in der Datenbank 30 enthaltenen Referenz-Spektren 14 nach deren Abfrage 38 und den in den Tabellen vorhandenen Tabellenparametern der Datensätze 15 des aktuellen Fingerabdrucks nach Übermittlung 48,
- Klassifizierung 45 der Vergleichsergebnisse in mit den Referenz-Spektren 14 übereinstimmende Fingerabdrücke und in mit den Referenz-Spektren 14 nicht übereinstimmende Fingerabdrücke, die als Fälschung definiert werden, als Klassifizierungsergebnisse sowie
- Übergabe 47 der Klassifizierungsergebnisse zur Anzeige 46.

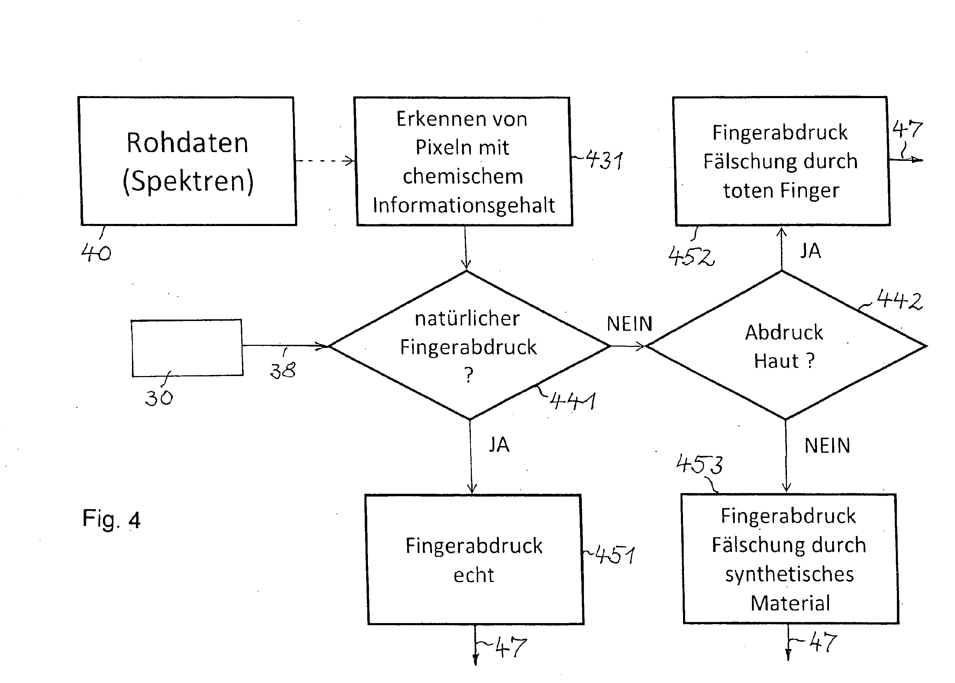

In Fig. 4 ist eine Darstellung der Endphase der Verfahrensschritte des Vergleiches 44 und der Klassifizierung 45 eines aktuellen Fingerabdrucks gezeigt. Die Endphase enthält folgende detaillierte Schritte - auch Unterschritte - in Verbindung mit den vorab genannten hauptsächlichen Schritten:
- Unterschritt: Erkennen 431 von Pixeln der aus der Tabelle der Funktionseinheit 34 bereitgestellten Pixel mit chemischem Informationsgehalt über die zwischenliegenden bearbeiteten hauptsächlichen Schritte 41, 42, 43,
- Unterschritte: Abarbeitung eines in die Vergleichseinheit 35 eingebundenen ersten Vergleichsalgorithmus 441 zur Feststellung eines aktuellen Fingerabdrucks nach Abfrage 38 von Referenz-Spektren 14 und Abarbeitung eines mit dem ersten Vergleichsalgorithmus 441 in Verbindung stehenden zweiten Vergleichsalgorithmus 442 zur Materialunterschiedsfeststellung des aktuellen Fingerabdrucks mit folgenden
- Unterschritten:
   - erste Entscheidung 451 auf Echtheit des aktuellen Fingerabdrucks,
   - zweite Entscheidung 452 auf Fälschung des aktuellen Fingerabdrucks und
   - dritte Entscheidung 453 auf Materialfälschung am aktuellen Fingerabdruck,
sowie
- Schritt:
   Übertragung 47 einer der zutreffenden Entscheidungen zur Anzeigeeinheit 12.

Die Auswertung der molekülspektroskopischen Bildgebung beruht auf den registrierten Absorptionsbanden aus den aufgenommenen Molekülspektren-Verläufen, insbesondere aus Extinktionsverläufen E(v), wobei die Extinktionsverläufe E(v) auf der Ausbildung des evaneszenten elektromagnetischen Feldes 3 und dessen Absorption in Abhängigkeit von der vorhandenen biochemischen Struktur der Zellen des Abdruckes geprägt werden, wobei das evaneszente elektromagnetische Feld 3 durch die Totalreflexion an der Auflagefläche 21 des Primas 2 entsteht.

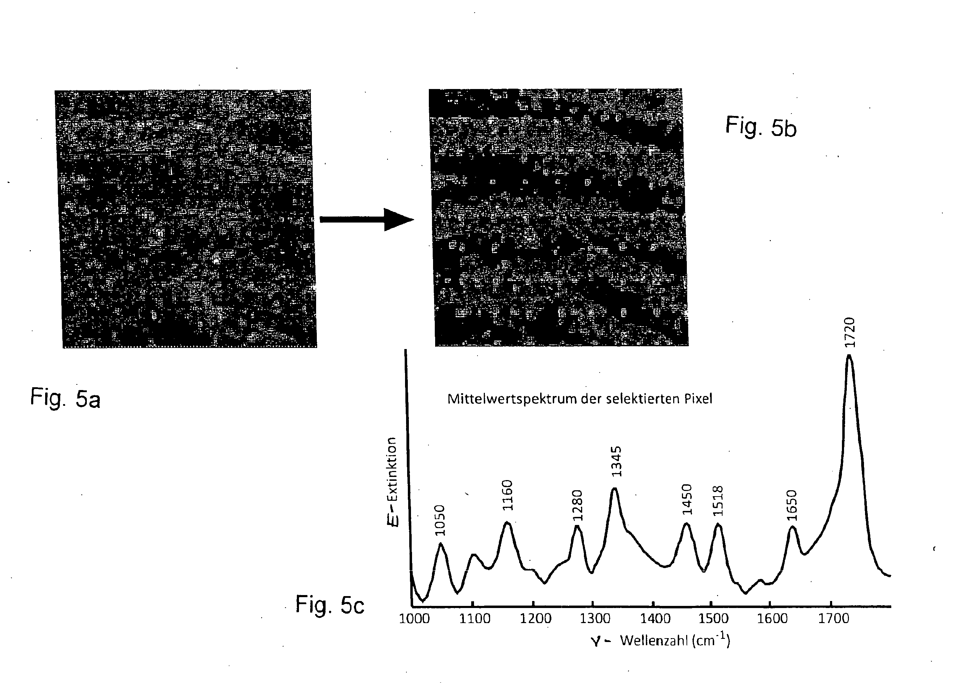

In der Fig. 5 ist eine Bearbeitungsfolge der aufgenommenen Rohdaten für die Referenz-Spektren 14 und Datensätze 15, wobei Fig. 5a eine Darstellung von Rohdaten der molekülspektroskopischen Bildgebung, wobei die angegebenen Grauwerte eine entsprechende integrable Extinktion darstellen. Die Fig. 5b ist eine Darstellung der molekülspektroskopischen Bildgebung von vorbehandelten Daten und entfernten Störungen durch Eliminierung von Pixeln ohne molekülspektroskopische Informationen (schwarz gefärbt) und durch Entfernen von Artefakten/Störungen (mit Basislinie, Rauschen unterdrücken und Eliminierung von Ausreißern) sowie Fig. 5c eine Extinktions(E)-Wellenzahl(v)-Kurve des Mittelspektrums der selektierten Pixel mit wellenzahlhervorgehobenen Piks zeigen,

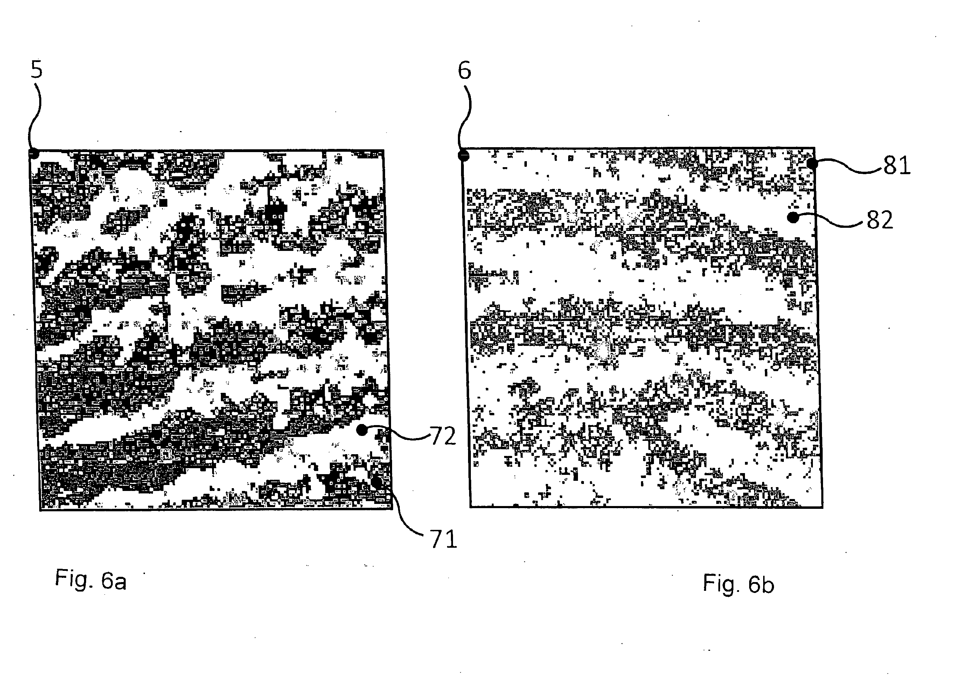

In Fig. 6 sind Ausschnitte (Vergrößerungen von etwa 4 x 4 mm²) von Fingerabdrücken angegeben, wobei Fig. 6a einen Ausschnitt des Fingerabdrucks der Fingerbeere eines toten Fingers und Fig. 6b einen Ausschnitt des Fingerabdruck der Fingerbeere eines lebenden Fingers zeigen. Zu sehen sind in Fig. 6a chemische molekülspektroskopische Signale 71 sowie Rillen 72, wobei letztere kein chemisches Signal darstellen. In Fig. 6b sind biochemische molekülspektroskopische Signale 81 sowie Rillen 82 zu erkennen.

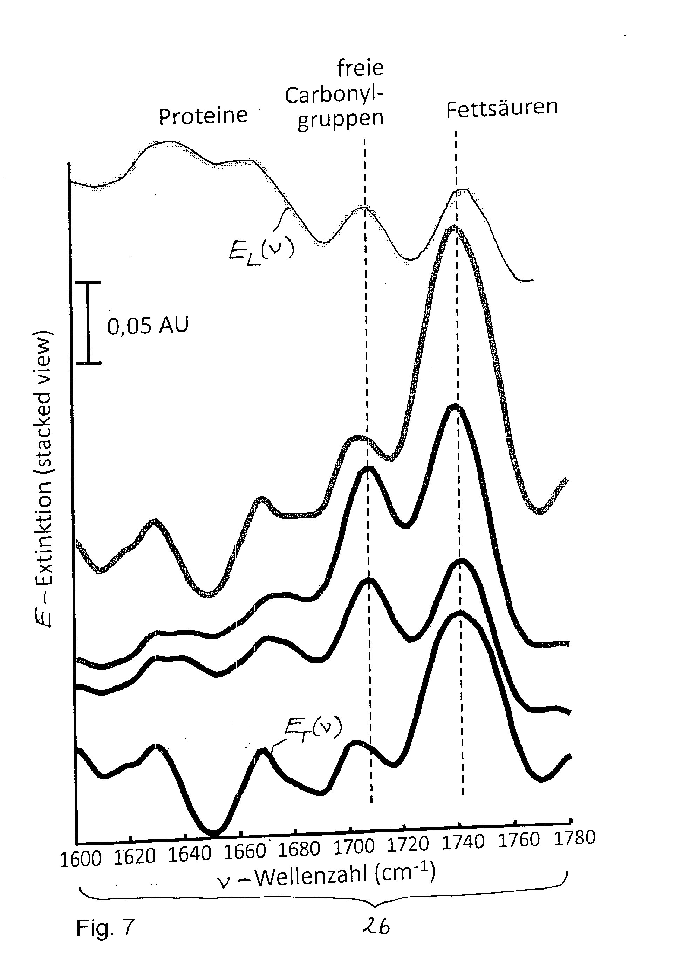

In Fig. 7 sind als Beispiel für die aufgenommenen Molekülspektren Extinktions(E)-Wellenzahl(v)-Kurven im Wellenzahl-Bereich 1600 cm⁻¹ und 1780 cm⁻¹ in Bezug auf Proteine, freie Carbonylgruppen und Fettsäuren der Zellen von verschiedenen Fingerabdrücken mit intakten (lebenden) Zellen, abgestuft teilgeschädigten Zellen oder total geschädigten (toten) Zellen dargestellt. Dabei ist die Kurve E_{L}(v) einem Fingerabdruck mit intakten (lebenden) Zellen, während die Kurve E_{T}(v) einem Fingerabdruck mit toten Zellen zugeordnet ist.
Der Unterschied zwischen lebenden Zellen und toten Zellen wird durch die unterschiedlichen E(v)-Kurven, insbesondere den spektralen Mustern der Proteine, der Carbonylgruppen und Fettsäuren durch deren Spaltung und Abbau ersichtlich. Insbesondere der Proteinabbau im Wellenzahl-Bereich zwischen 1630 cm⁻¹ und 1670 cm⁻¹ gibt ein Indiz für die toten Zellen des aktuellen Fingerabdrucks. Der Fettsäurenabbau bei toten Zellen einer Fingerbeere 1 ist größer als bei lebenden Zellen. Ebenso sind die Abbauprodukte der Carbonylgruppen bei toten Fingern größer als bei lebenden Zellen einer Fingerbeere 1.

Im Unterschied zum Stand der Technik wird in dem erfindungsgemäßen Verfahren und der zugehörigen Anordnung keine Blutzellenänderungen, sondern die Zusammensetzung der Haut der Zellen als Vergleichsobjekt eingesetzt.
Mit der infrarot-molekülspektroskopischen Bildgebung der Zellen der Haut sind die Infrarot-Spektren präziser erkennbar als die verschiedenen Zustände der Blutzellen in der Haut gemäß dem Stand der Technik.

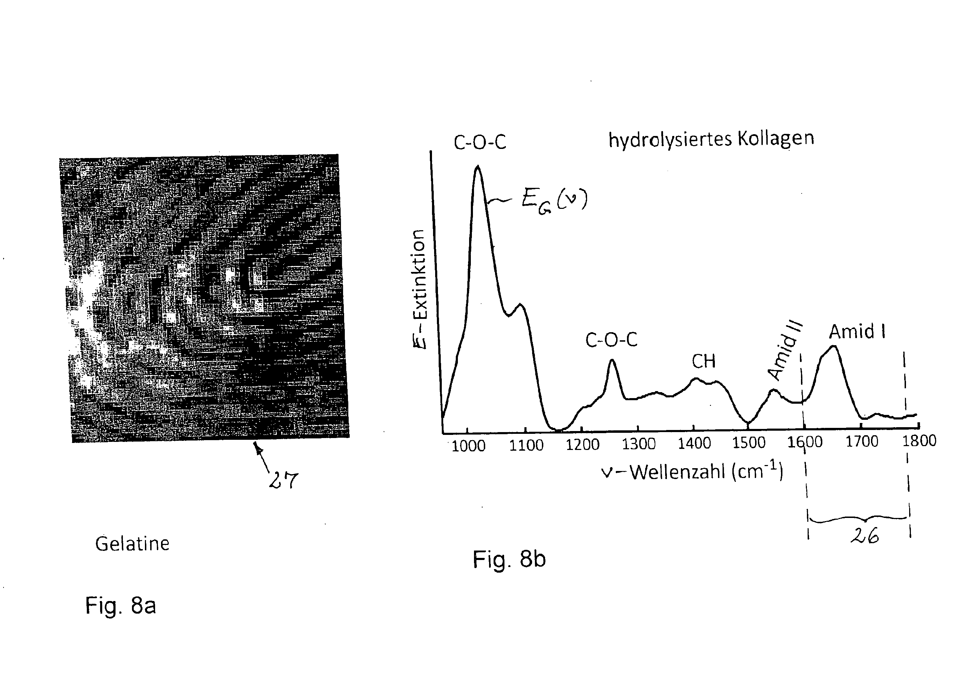

In Fig. 8 ist ein erstes Materialfälschungs-Beispiel der Anzeige für ein Gelatine-Fingerabdruckmuster in Bezug auf ein ausgewertetes Gelatine-Rillenmuster 27 und auf eine ausgewertete molekülspektroskopische Bildgebung, wobei Fig. 8a den zugehörigen gefälschten Fingerabdruck aus getrockneter Gelatine mit Rillenmuster 27 und Fig. 8b die zugehörige ausgewertete Extinktions(E)-Wellenzahl(v)-Kurve E_{G}(V) für die einen Fingerabdruck darstellenden, getrockneten Gelatine zeigen.
Bei Vergleich der E_{G}(v)-Kurve im Wellenzahl-Bereich 26 zwischen 1600 cm⁻¹ und 1780 cm⁻¹ und der E_{L}(v)-Kurve eines echten (lebenden) Fingerabdrucks zeigt keine Übereinstimmung. Es liegt also eine Fälschung vor.

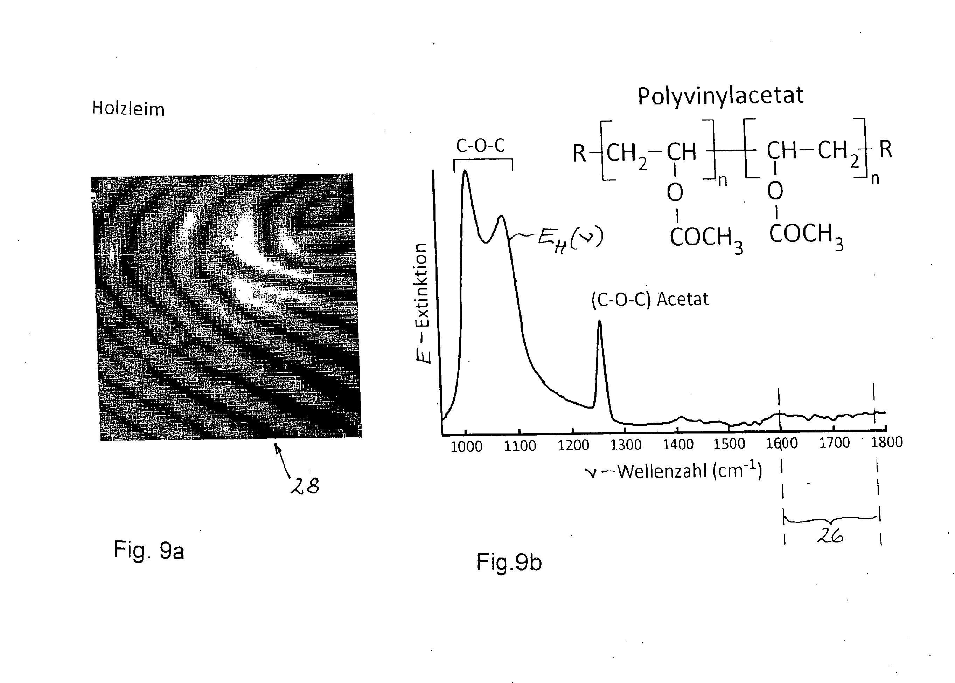

In Fig. 9 ist ein zweites Materialfälschungs-Beispiel der Anzeige für ein Holzleim-Fingerabdruckmuster in Bezug auf ein ausgewertetes Holzleim-Rillenmuster 28 und auf eine ausgewertete molekülspektroskopische Bildgebung dargestellt, wobei Fig. 9a den zugehörigen gefälschten Fingerabdruck aus getrocknetem Holzleim mit Rillenmuster 28 und Fig. 9b die zugehörige ausgewertete Extinktions(E)-Wellenzahl(v)-Kurve E_{H}(v) für den einen Fingerabdruck darstellenden, getrockneten Holzleim zeigen.
Bei Vergleich der E_{H}(v)-Kurve des Holzleim-Fingerabdruckmusters im Wellenzahl-Bereich 26 zwischen 1600 cm⁻¹ und 1780 cm⁻¹ und der E_{L}(v)-Kurve eines echten (lebenden) Fingerabdrucks zeigt keine Übereinstimmung. Es liegt also eine Fälschung vor.

Die in der Datenbank 30 vorhandenen Referenz-Spektren 14 können nicht nur die Vorab-Fingerabdrücke mit der Haut von lebenden Fingern 1, sondern auch Vorab-Fingerabdrücke von teilgeschädigten und totalgeschädigten Hautzellen der Fingerbeeren sowie auch die Vorab-Abdrücke in Materialen mit nachgeahmten künstlichen Fingerabdrücken (wie bei Gelatine oder Holzleim) oder auch Vorab-Abdrücke von realen Fingerabdrücken auf Materialien (Leder, Textilien, Metall) oder von unbedruckten Materialien sein.

Mit der Erfindung wird gezeigt,
dass eine Unterscheidung z.B. von künstlichen Fingerabdrücken mittels Infrarot-Bildgebungs-Molekülspektroskopie sehr gut möglich ist und
dass auch eine Unterscheidung von Fingerabdrücken mit lebenden Zellen und/oder toten Zellen ebenfalls molekülspektroskopisch möglich ist.

### Bezugszeichenliste

- 1: aufgelegte Fingerbeere
- 2: optisches Prisma
- 2a: einfallendes Licht
- 2b: reflektiertes Licht
- 2c: transmittiertes Licht
- 3: evaneszentes elektromagnetisches Feld
- 3a: einfallendes Lichtbündel
- 3b: totalreflektiertes Lichtbündel
- 4: Arraydetektor für Infrarotlicht
- 41: Kamerasensor nach dem Stand der Technik
- 5: Ausschnitt eines Fingerabdrucks eines toten Fingers
- 6: Ausschnitt eines Fingerabdrucks eines lebenden Fingers
- 10: Auswerteeinheit
- 101: Auswerteeinheit nach dem Stand der Technik
- 11: Fingerabdruck-Aufnahmeeinheit
- 12: Anzeigeeinheit
- 13: Beleuchtungseinrichtung
- 14: Referenz-Spektren
- 15: Datensätze des aktuellen Fingerabdrucks
- 131: Beleuchtungseinrichtung nach dem Stand der Technik
- 20: erfindungsgemäße Vorrichtung
- 21: Auflagefläche/Grundfläche
- 22: erste Seitenfläche
- 23: zweite Seitenfläche
- 24: Auflagefläche nach dem Stand der Technik
- 25: Prismenkörper nach dem Stand der Technik
- 26: Vergleichs-Wellenzahl-Bereich
- 27: Gelatine-Rillenmuster
- 28: Holzleim-Rillenmuster
- 29: Vorab-Speicherung
- 30: Datenbank für Referenz-Spektren
- 31: Speichereinheit mit Datensätzen für selektive Spektren
- 32: erste Funktionseinheit zur Datenvorbehandlung
- 33: zweite Funktionseinheit zur multivariaten Datenanalyse
- 34: dritte Funktionseinheit zur Speicherung von Tabellenparametern
- 35: Vergleichseinheit
- 36: Klassifizierungseinheit
- 37: Zuleitung für zu speichernde Referenz-Spektren
- 38: Abfrageleitung
- 39: Signalleitung
- 40: Schritt: Speicherung
- 41: Schritt: Datenvorbehandlung
- 42: Schritt: Datenanalyse
- 43: Schritt: Speicherung von Tabellen
- 431: Unterschritt: Erkennen von Pixeln mit chemischem Informationengehalt
- 44: Schritt: Vergleich
- 441: Unterschritt: Vergleichsalgorithmus-Durchführung für Fingerhaut
- 442: Unterschritt: Vergleichsalgorithmus-Durchführung für von Fingerhaut abweichendem Material
- 45: Schritt: Klassifizierung
- 451: Unterschritt: erste Entscheidung eines echten Fingerabdrucks
- 452: Unterschritt: zweite Entscheidung eines unechten Fingerabdrucks
- 453: Unterschritt: dritte Entscheidung eines Materialfälschungs-Abdrucks
- 46: Schritt: Anzeige
- 47: Schritt: Zuführung von Klassifizierungsdaten zur Anzeige
- 48: Schritt: Übergabe von chemischen Informationen
- 71: chemisches, spektroskopisches Signal
- 72: Rillen (kein chemisches Signal)
- 81: biochemisches, spektroskopisches Signal
- 82: Rillen (kein biochemisches Signal)

## Patentansprüche

1. Anordnung (20) zur Identifizierung der Echtheit von Fingerabdrücken, aufweisend eine an eine datenverarbeitende Auswerteeinheit (10) angeschlossene Fingerabdruck-Aufnahmeeinheit (11), die mit einer Auflagefläche (21) und mit Echtzeiterkennung für eine auf die Fingerabdruck-Aufnahmeeinheit (11) aufgelegte Fingerbeere (1) versehen ist, wobei zur Identifizierung und Echtzeiterkennung eine ortsaufgelöste spektroskopische Reflexionsmessung im Bereich von 4000 bis 500 Wellenzahlen durchgeführt wird, wobei der Fingerabdruck-Aufnahmeeinheit (11) ein Sensor (4) nachgeordnet ist, um von der auf der Auflagefläche (21), die durch eine Fläche eines Prismenkörpers (2) gebildet wird, aufgelegten Fingerbeere (1) abgelenktes Licht (3b) einer Beleuchtungseinrichtung (13) aufzunehmen, wobei der Sensor (4) signaltechnisch mit der Auswerteeinheit (10) verbunden ist, die mit einer nachgeordneten Anzeigeeinheit (12) in Verbindung steht,
wobei der Prismenkörper (2) ein Prisma darstellt, dessen Grundfläche die Auflagefläche (21) für die Fingerbeere (1) ist und dessen eine geneigte Seitenfläche (22) die Eintrittsfläche für das einfallende infrarote Lichtbündel (3a) der Beleuchtungseinrichtung (13) und dessen andere geneigte Seitenfläche (23) die Austrittsfläche für das austretende infrarote Lichtbündel (3b) darstellen, wobei das einfallende und innenseitig des Prismas (2) auf die Grundfläche (21) auftreffende Lichtbündel (3a) und die Normale der Grundfläche (21) im Bereich eines Totalreflexionswinkels (α_{T}) liegen, wodurch das totalreflektierende Lichtbündel (3b) außenseitig des Prismas (2) auf der Grundfläche (21) ein evaneszentes elektromagnetisches Feld (3) ausbildet, das mit dem Abdruck der aufliegenden Fingerbeere (1) in Kontakt tritt und zellmolekularabhängig einer Absorption unterliegt, und danach als totalreflektiertes Lichtbündel (3b) auf einen den Sensor darstellenden Arraydetektor (4) zur spektroskopischen Bildgebung geleitet wird,
**dadurch gekennzeichnet,**
**dass** der Bildkontrast auf der molekularen Zusammensetzung der Fingerbeere beruht, wobei die molekülspektroskopische Bildgebung auf den registrierten Absorptionsbereichen aus den aufgenommenen Molekülspektren basiert, wobei die Molekülspektren von der Ausbildung des evaneszenten elektromagnetischen Feldes (3) und dessen Absorption in Abhängigkeit von der vorhandenen molekularen Struktur der Zellen geprägt werden,
wobei in der Auswerteeinheit (10) die Molekülspektren im Wellenzahl-Bereich zwischen 1600cm⁻¹ und 1780cm⁻¹ auf Proteine, freie Carbonylgruppen und Fettsäuren der Zellen von verschiedenen Fingerabdrücken bezogen und verglichen werden, wobei aus dem Vergleich der unterschiedlichen spektralen Muster der Proteine, der Carbonylgruppen und Fettsäuren und aus deren Spaltung und Abbau Indizien zur Unterscheidung der Fingerabdrücke gegeben sind, die durch den Proteinabbau, den Fettsäurenabbau und die Abbauprodukte der Carbonylgruppen unterstützt werden, wobei die spektralen Muster die Gesamtheit der Absorptionssignale einer Stoffgruppe oder Molekülgruppe darstellen, und
wobei die in der Auswerteeinheit (10) durchgeführte molekülspektroskopische Auswertung des abgeschwächten totalreflektierten infraroten Lichtbündels (3b) die Bewertung der biochemischen Zusammensetzung des Fingerabdruckes gestattet,
und wobei die Auswerteeinheit (10) zumindest folgende Funktionseinheiten, in der folgenden Reihenfolge auch hardwaremäßig miteinander verbunden, enthält:
- eine erste Funktionseinheit (32) zur Datenvorbehandlung und Entfernen von Störungen,
- eine zweite Funktionseinheit (33) für eine multivariate Datenanalyse und Ermittlung in einem signifikanten zellmolekularabhängigen Absorptionsbereich mit dem Wellenzahlbereich zwischen 1600cm⁻¹ und 1780cm⁻¹,
- eine dritte Funktionseinheit (34) zur Speicherung einer Tabelle mit dem signifikanten Absorptionsbereich mit dem Wellenzahlbereich zwischen 1600cm⁻¹ und 1780cm⁻¹, mit dessen Position und mit dessen Intensität als Tabellenparameter,
- eine Vergleichseinheit (35) zum Vergleich der Datensätze (15) des aktuellen Fingerabdrucks mit den abgerufenen gespeicherten Referenz-Spektren (14) im Wellenzahlbereich zwischen 1600cm⁻¹ und 1780cm⁻¹ aus der Datenbank (30) und zur Erzeugung von Vergleichsergebnissen,
- eine Klassifizierungseinheit (36) zur Klassifizierung der weitergeleiteten Vergleichsergebnisse mit Klassifizierungsergebnissen als identifizierter übereinstimmender Fingerabdruck oder als Fälschung und zur Übertragung der Klassifizierungsergebnisse an die der Auswerteeinheit (10) nachgeordnete Anzeigeeinheit (12).

2. Anordnung nach Anspruch 1,
**dadurch gekennzeichnet,**
**dass** die Molekülspektren in Form von Extinktions(E)-Wellenzahl(v)-Kurven (E(v)) oder Transmissionsspektren oder Absorptionsspektren ausgebildet sind.

3. Anordnung nach Anspruch 1,
**dadurch gekennzeichnet,**
**dass** der dem Arraydetektor (4) angeschlossenen Auswerteinheit (10) eine Datenbank (30), in der Referenz-Spektren (14) gespeichert sind und die zu Vergleichen mit Datensätzen (15) eines aktuellen Fingerabdrucks zur Verfügung stehen, und eine Speichereinheit (3), die für die Speicherung der Datensätze (15) von selektierten Spektren des aktuellen Fingerabdrucks der Fingerbeeren (1) dient, zugeordnet sind, wobei die Datensätze (15) eines aktuellen Fingerabdrucks die biochemischen Informationen und die Zuordnung zur örtlichen Position des zugehörigen Rillenmusters enthalten.

4. Anordnung nach den Ansprüchen 1 bis 3,
**dadurch gekennzeichnet,**
**dass** die Speichereinheit (31) und die in der Auswerteeinheit (10) enthaltenen Funktionseinheiten (32,33,34) sowie die Vergleichseinheit (35) und die Klassifizierungseinheit (36) der Auswerteeinheit (10) Hardware-Baugruppen sind, die zumindest mit funktionsbedingten und -bezogenen programmtechnischen Mitteln versehen sind und untereinander in signaltechnischer und programmtechnischer Verbindung stehen.

5. Anordnung nach den Ansprüchen 1 bis 4,
**dadurch gekennzeichnet,**
**dass** die in der Datenbank (30) gespeicherten Referenz-Spektren (14) von
- Vorab-Fingerabdrücken mit der Haut von Fingerbeeren (1) lebender Finger,
- Vorab-Fingerabdrücken von teilgeschädigten und totalgeschädigten Hautzellen der Fingerbeeren (1),
- Vorab-Abdrücken in Materialen mit nachgeahmten künstlichen Fingerabdrücken sowie
- Vorab-Abdrücken von realen Fingerabdrücken auf Materialien oder
- unbedruckten Materialien
sind.

6. Verfahren zur Identifizierung der Echtheit von Fingerabdrücken, aufweisend eine an eine datenverarbeitende Auswerteeinheit (10) angeschlossene Fingerabdruck-Aufnahmeeinheit (11), die mit einer Auflagefläche (21) und mit Echtzeiterkennung für eine auf die Fingerabdruck-Aufnahmeeinheit (11) aufgelegte Fingerbeere (1) versehen ist, wobei zur Identifizierung und Echtzeiterkennung eine ortsaufgelöste spektroskopische Reflexionsmessung im Bereich von 4000 bis 500 Wellenzahlen durchgeführt wird, wobei der Fingerabdruck-Aufnahmeeinheit (11) ein Sensor (4) nachgeordnet ist, um von der auf der Auflagefläche (21), die durch eine Fläche eines Prismenkörpers (2) gebildet wird, aufgelegten Fingerbeere (1) abgelenktes Licht (3b) einer Beleuchtungseinrichtung (13) aufzunehmen, wobei der Sensor (4) signaltechnisch mit der Auswerteeinheit (10) verbunden ist, die mit einer nachgeordneten Anzeigeeinheit (12) in Verbindung steht, unter Einsatz einer Anordnung nach den Ansprüchen 1 bis 5,
gekennzeichnet zumindest durch folgende hauptsächliche Schritte:
- Vorab-Speicherung (29) von selektierten Referenz-Spektren (14) in einer Datenbank (30) und
während und nach Aufnahme des aktuellen Fingerabdrucks in dieser Reihenfolge,
- Speicherung (40) von selektierten Spektren eines aktuellen Fingerabdrucks als Datensätze (15) in einer Speichereinheit (31),
- Datenvorbehandlung (41) und Entfernen von Störungen in den Datensätzen (15) des aktuellen Fingerabdrucks aus der Speichereinheit (31),
- Multivariate Datenanalyse (42) und Ermitteln von signifikanten Absorptionsbereichen aus den Datensätzen (15) des aktuellen Fingerabdrucks,
- Speicherung (43) von Tabellen mit den signifikanten Absorptionsbereichen, deren Positionen und deren Intensitäten als Tabellenparameter,
- Vergleich (44) zwischen den in der Datenbank (30) enthaltenen Referenz-Spektren (14) nach deren Abfrage (38) und den in den Tabellen vorhandenen Tabellenparametern der Datensätze (15) des aktuellen Fingerabdrucks nach Übermittlung (48),
- Klassifizierung (45) der Vergleichsergebnisse in mit den Referenz-Spektren (14) übereinstimmende Fingerabdrücke und in mit den Referenz-Spektren (14) nicht übereinstimmende Fingerabdrücke, die als Fälschung definiert werden, als Klassifizierungsergebnisse sowie
- Übergabe (47) der Klassifizierungsergebnisse als spektroskopische Rillenmuster und Molekülspektren zur Anzeige (46),
wobei die Molekülspektren im Wellenzahl-Bereich zwischen 1600cm⁻¹ und 1780cm⁻¹ auf Proteine, freie Carbonylgruppen und Fettsäuren der Zellen von verschiedenen Fingerabdrücken verglichen werden, wobei der Unterschied zwischen verschiedenen Zellen durch die unterschiedlichen spektralen Muster der Proteine, der Carbonylgruppen und Fettsäuren durch deren Spaltung und Abbau gegeben sind und wobei die spektralen Muster die Gesamtheit der Absorptionssignale einer Stoffgruppe oder Molekülgruppe darstellen.

7. Verfahren nach Anspruch 6,
**dadurch gekennzeichnet,**
**dass** in der Endphase der Verfahrensschritte bei dem Vergleich (44) und bei der Klassifizierung (45) des aktuellen Fingerabdrucks folgende detaillierte Unterschritte in Verbindung mit den hauptsächlichen Schritten vorgesehen werden:
- Unterschritt:
Erkennen (431) von Pixeln der aus der Tabelle der Funktionseinheit (34) bereitgestellten Pixel mit chemischem Informationsgehalt über die zwischenliegenden bearbeiteten hauptsächliche Schritte (41, 42, 43),
- Unterschritte:
Abarbeitung eines in die Vergleichseinheit (35) eingebundenen ersten Vergleichsalgorithmus (441) zur Feststellung eines aktuellen Fingerabdrucks nach Abfrage (38) von Referenz-Spektren (14) und Abarbeitung eines mit dem ersten Vergleichsalgorithmus (441) in Verbindung stehenden zweiten Vergleichsalgorithmus (442) zur Materialunterschiedsfeststellung des aktuellen Fingerabdrucks mit folgenden
- Unterschritten:
- erste Entscheidung (451) auf Echtheit des aktuellen Fingerabdrucks,
- zweite Entscheidung (452) auf Fälschung des aktuellen Fingerabdrucks und
- dritte Entscheidung (453) auf Materialfälschung am aktuellen Fingerabdruck
sowie
- Schritt:
Übertragung (47) eines der zutreffenden Entscheidungsergebnisse zur Anzeigeeinheit (12).

8. Verfahren nach den Ansprüchen 6 und 7,
**dadurch gekennzeichnet,**
**dass** das evaneszente elektromagnetische Feld (3) durch die Totalreflexion an der Auflagefläche (21) des Primas (2) entsteht, wobei als Absorptionsbereiche Absorptionsbanden als solche, Kanten, Flanken, Schultern von Absorptionsbanden oder zusammengesetzte Absorptionsbanden gelten.

9. Verfahren nach den Ansprüchen 6 bis 8,
**dadurch gekennzeichnet,**
**dass** die Molekülspektren auf Proteine, freie Carbonylgruppen und Fettsäuren der Zellen von verschiedenen Fingerabdrücken mit intakten (lebenden) Zellen, abgestuft teilgeschädigten Zellen oder total geschädigten (toten) Zellen bezogen werden, wobei der Unterschied zwischen lebenden Zellen und toten Zellen durch die unterschiedlichen spektralen Muster der Proteine, der Carbonylgruppen und Fettsäuren durch deren Spaltung und Abbau gegeben sind, wobei der Proteinabbau ein Indiz für die toten Zellen des aktuellen Fingerabdrucks gibt, wobei der Fettsäurenabbau bei toten Zellen einer Fingerbeere (1) größer als bei lebenden Zellen ist und ebenso die Abbauprodukte der Carbonylgruppen bei toten Fingern größer als bei lebenden Zellen einer Fingerbeere (1) sind, wobei die spektralen Muster die Gesamtheit der Absorptionssignale einer Stoffgruppe oder Molekülgruppe darstellen.

10. Verfahren nach den Ansprüchen 6 bis 9,
**dadurch gekennzeichnet,**
**dass** die Molekülspektren in Form von Extinktions(E)-Wellenzahl(v)-Kurven (E(v)) oder Transmissionsspektren oder Absorptionsspektren ausgebildet werden.
